(19) **Europäisches Patentamt**
**European Patent Office**
**Office européen des brevets**

(11) **EP 4 574 052 A1**

(12) **EUROPEAN PATENT APPLICATION**

(43) Date of publication:
**25.06.2025 Bulletin 2025/26**

(21) Application number: **23219558.6**

(22) Date of filing: **21.12.2023**

(51) International Patent Classification (IPC):
***A61B 8/08*** (2006.01)    ***G01S 7/52*** (2006.01)
***G06T 7/00*** (2017.01)

(52) Cooperative Patent Classification (CPC):
**A61B 8/485; A61B 8/08; A61B 8/085; A61B 8/481;**
**A61B 8/5223; G01S 7/52042; G06T 7/0016**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **NanoEcho AB**
**222 29 Lund (SE)**

(72) Inventors:
• **Bergh, Daniel**
  **247 52 Dalby (SE)**
• **Haner, Sebastian**
  **411 23 Göteborg (SE)**
• **Santesson, Magnus**
  **246 32 Löddeköpinge (SE)**

(74) Representative: **KIPA AB**
**Drottninggatan 11**
**252 21 Helsingborg (SE)**

(54) **ALGORITHM FOR DETERMINING TISSUE DISPLACEMENT IN ULTRASOUND IMAGING**

(57)    A method and system for estimating at least one displacement parameter, characterizing a displacement signal of a medium in a sequence of ultrasound data frames.

**Fig. 4**

## Description

### Field of the Invention

This invention pertains in general to the field of ultrasound imaging, such as magnetomotive imaging or elastography. In particular, the invention relates to an algorithm for determining tissue displacement in ultrasound imaging, such as a real-time algorithm. In more particular, the real-time algorithm makes it possible to observe oscillating movement induced by the nanoparticles in tissue when exposed to an alternating magnetic field.

### Background of the Invention

Magnetomotive ultrasound imaging utilizes iron-oxide-based nanoparticles as a contrast agent. For example to indicate lymph nodes close to a tumour and provide an indication of whether they contain metastases. By means of an alternating magnetic field, generated within a magnetomotive probe, such as a handheld probe, the particles are set in motion. Thus, a vibration appears in the tissue where particles are present. The vibration is detected with ultrasound and processed by the software, which filters, enhances and then visualizes the localization of the vibration in an image.

The amplitude of the oscillation is typically very small - on the scale of micrometers, see for instance Maria Evertsson, et al "Multimodal detection of iron oxide nanoparticles in rat lymph nodes using magnetomotive ultrasound imaging and magnetic resonance imaging" IEEE transactions on ultrasonics, ferroelectrics, and frequency control, 61 (8):1276-1283, 2014, and Evertsson M, et al "Frequency- and phase-sensitive magnetomotive ultrasound imaging of superparamagnetic iron oxide nanoparticles" IEEE Trans Ultrason Ferroelectr Freq Control. 2013 Mar;60(3):481-91. doi: 10.1109/TUFFC.2013.2591. PMID: 23475915.

This makes the movement practically impossible to observe in a standard B-mode ultrasound image. Instead, one seeks to construct an image where each pixel encodes the amplitude and/or phase of the oscillating movement at the corresponding location.

The simplest and most studied technique for estimating complex amplitude given RF data is described in, for example, Maria Evertsson, "Development of Magnetomotive Ultrasound Imaging", PhD thesis, Lund University, 2016; Sandra Sjöstrand, et al, "Magnetomotive ultrasound imaging systems: basic principles and first applications", Ultrasound in Medicine & Biology, 46(10):2636-2650, 2020; and Maria Holst, et al, "Phase-locked magnetomotive ultrasound imaging of superparamagnetic iron-oxidenanoparticles", 2010 IEEE International Ultrasonics Symposium, pages 1007-1010. IEEE, 2010.

Instead of estimating displacement from the radiofrequency (RF) data directly, this method is based on analysis of the IQ data. The term IQ is an abbreviation for "in-phase" and "quadrature". The RF data is initially converted to IQ data using quadrature demodulation, i.e. by multiplying the RF signal with two sinusoids shifted by 90 degrees relative one another. By interpreting the two signals I and Q as describing a single complex valued function, the signal used for estimating displacement is now taken as the argument (or phase) of this IQ signal.

The major drawback with this method is that the phase of the IQ signal tends to be noisy. To compensate for this, one needs a large number of frames, typically hundreds, to estimate the complex amplitude and obtain a stable image. This results in high amounts of latency, which makes the method inherently hard to use together with for example hand-held ultrasound devices.

There are also less noise sensetive methods described in the literature. In Thomas Ersepke, et al, "On the performance of time domain displacement estimators for magnetomotive ultrasound imaging. IEEE transactions on ultrasonics, ferroelectrics, and frequency control", 66(5):911-921, 2019, several techniques for estimating displacement between pairwise frames are described and evaluated.

However, even further improvements to make the method more stable and further reduce noise to the displacement signal would be an advantage. In particular for handheld magnetomotive probes.

### Summary of the Invention

Accordingly, examples of the present invention preferably seek to mitigate, alleviate or eliminate one or more deficiencies, disadvantages or issues in the art, such as the above-identified, singly or in any combination by providing a method, computer program and system for establishing displacements, such as movements, in ultrasound imaging. In particular, the disclosed technique may be applied for magnetomotive imaging.

In one disclosure, a method for estimating at least one displacement parameter, characterizing a displacement signal of a medium in a sequence of ultrasound data frames is described. The method may include acquiring a first batch of data frames using an ultrasound device. The method may also include inputting the first batch of data frames into a displacement parameter estimating algorithm together with an estimated previous state to obtain an estimated next state, the estimated next state comprising an amplitude and a phase, and wherein the estimated next state is used as the estimated previous state for a second batch of data frames. The method may also include associating the at least one displacement parameter estimated by the estimating algorithm with at least one of the amplitude and the phase, and using the at least one displacement parameter to localize movement in the medium.

In some examples of the method, a fitting model is used to localize the movement in the medium, such as a sinusoidal fitting model.

In some examples of the method, wherein the ultrasound device is part of a Magnetomotive ultrasound imaging device and/or elastography device.

In some examples of the method, wherein the amplitude and the phase are represented by a complex amplitude.

In some examples of the method, coding the phase and the amplitude estimated by the estimation algorithm using a heat map and scan conversion to provide an output image.

In some examples of the method, wherein a precision matrix is obtained for determining the significance of the previous state.

In some examples of the method, wherein a precision matrix is used for estimating the significance of a displacement estimation; or wherein a precision matrix is used for estimating the significance of a displacement estimation being a displacements estimation at the location of an image pixel, wherein these displacements with significance values lower than a pre-defined threshold is set to zero, optionally, the pixels set to zero displays a grayscale B-mode value.

In some examples of the method, wherein the second batch of data frames comprises at least one data frame acquired after, such as subsequent, the data frames of the first batch, or a combination of at least one data frame of the first batch together with at least one data frame acquired after, such as subsequent, the data frames of the first batch.

In some examples of the method, wherein for the first batch of data frames, the estimated previous state comprises at least one displacement parameter set to a predefined value and the value representing the accuracy of the estimate of the at least one displacement parameter is set to define the accuracy as either high or low.

In some examples of the method, wherein the displacement represents movement of particles in magnetomotive ultrasound imaging.

In some examples of the method, wherein the data frames of the first and second batch are subsequently ordered.

In some examples of the method, wherein the displacement estimating algorithm comprises a pairwise displacement estimator and a recursive least-squares fitting for estimating the amplitude and the phase, and wherein the previous state together with an estimate of its accuracy is used as prior for each calculation using the displacement parameter estimating algorithm.

In some examples of the method, wherein the displacement estimating algorithm comprises an optical flow step and the amplitude and the phase is estimated directly from said first batch of data frames by including a time dependency in the optical flow step, and at every time step, the previous state together with an estimate of its accuracy is used as prior.

In some examples of the method, detecting interfering or artefactual motion by comparing the first batch with the second batch.

In some examples of the method, wherein the phase is used for estimating a location of the nanoparticles or providing information about tissue stiffness, such as a detected motion of tissue is interpreted as presence of nanoparticles and since the motion is transferred in tissue with a delay, the phase is different in regions not containing the nanoparticles, whereby the difference in phase is used for detecting the location of the nanoparticles, or estimating tissue stiffness. Furthermore, by identifying the location of the center of the displacement amplitude induced by the nanoparticles, knowledge of the transferred movement which gradually decays with distance from the center could be filtered out, further enhancing the precision in the localization of nanoparticles. Normally the location of the center of the displacement amplitude induced by the nanoparticles is the location of the highest amplitude. By identifying a local maximum in the displacement amplitude and consider as for example deducting the expected decay in displacement amplitude around the maximum which is caused by the spreading movement one can obtain a more precise image of the actual movement origin and thereby nanoparticle location.

In a further disclosure, a computer program including instructions which, when the program is executed by a computer, cause the computer to carry out any step of the method described above.

In yet another disclosure, a system for examining tissue, comprising:

a magnetomotive ultrasound device, and
a processing unit configured to execute the computer program of the method described above-.

In the context of this description:

RF Data means data collected from the transducer containing raw echo data samples from one or multiple (all) used ultrasound transducer elements. Typically data from several elements (called a sub-aperture) is used to create one ultrasound line. RF-Data can have been processed for example by doing filtering and/or decimation.

Beamformed Ultrasound data (RF or IQ) means Ultrasound RF data from several transducer elements that has been processed to represent data as coming from one ultrasound beam, in a process often referred to as beamforming. This data is the underlying data representing one image line, extending from the transducer. We therefore denote each data point in this image line an RF pixel, as each line may be put next to another to represent an image, though somewhat geometrically distorted.

Ultrasound data frame means RF Data frame or Beamformed RF frame or IQ data frame or scan converted data frames or RF-derived data that comprises all data necessary to form a static image from an ultrasound scan. That is data from the transducer elements used to form the lines for a single image. The data used for the algorithm is a sequence of ultrasound data frames.

Some advantages with the described technique:
Real-time is within the context of this description used to describe an actual time during which something takes place. For example, an algorithm that includes operations in computing or other processes that must guarantee response times within a specified time, usually a relatively short time. A real-time process is generally one that happens in defined time steps of maximum duration and fast enough to affect the environment in which it occurs, such as images timely updated during examination or visualization. A short time period that allows for a timely update during examination or visualization should be so short that a user considers it to be sufficiently immediate, i.e. there is no delay or lag that will affect the examination or visualization. Real time within this context is normally within the time range of about a second or less, such as milliseconds, such as 1ms to 1000ms, such as 1ms to 500ms, such as 1ms to 100ms, such as 1 to 50ms.

Further embodiments of the invention are defined in the dependent claims, wherein features for the second and subsequent aspects of the invention are as for the first aspect mutatis mutandis.

It should be emphasized that the term "comprises/comprising" when used in this specification is taken to specify the presence of stated features, integers, steps or components but does not preclude the presence or addition of one or more other features, integers, steps, components or groups thereof.

## Brief Description of the Drawings

These and other aspects, features and advantages of which embodiments of the invention are capable of will be apparent and elucidated from the following description of embodiments of the present invention, reference being made to the accompanying drawings, in which:

Figs. 1 is illustrating a schematic magnetomotive imaging probe assembly according to an example of the present disclosure;
Figs.2 illustrates an exemplary flowchart of a phase-shift method for estimating displacement;
Fig. 3 is illustrating an exemplary flowchart of a two-step version of the described algorithm; and
Fig. 4 is illustrating an exemplary flowchart of a one-step version of the algorithm described herein.

## Description of examples

Specific examples of the disclosure will now be described with reference to the accompanying drawings. This disclosure may, however, be embodied in many different forms and should not be construed as limited to the examples set forth herein; rather, these examples are provided so that this disclosure will be thorough and complete, and will fully convey the scope of the disclosure to those skilled in the art. The terminology used in the detailed description of the examples illustrated in the accompanying drawings is not intended to be limiting of the disclosure. In the drawings, like numbers refer to like elements.

The following description focuses on an example of a method for an algorithm, such as a real-time algorithm, for determining tissue displacement in magnetomotive ultrasound imaging. The method may be applicable to data recorded using an magnetomotive ultrasound imaging probe. The method is in particular advantageous for visualization of tissue displacements in a non-stationary environment. For example, for handheld magnetomotive ultrasound imaging probes where the method may be useful to aid visualizing the tissue displacement which may be problematic otherwise due to movement of the probe. The method may also aid in visualizing the tissue displacement when the patient is moving. However, it will be appreciated that the disclosure is not limited to this application but may be applied to many other fields and applications. For instance, as an alternative to using particles set in motion using a magnet, tracking of equally minute tissue displacements may be ultrasound elastography, where tissue is set in motion using a focused ultrasound pulse, such as a push pulse, or using an external vibrator. The motion may then be imaged using an ultrasound sensor.

As an example, the push pulses may be one or multiple push pulses.

In magnetomotive imaging, a magnet is used to generate an inhomogeneous magnetic field. The inhomogeneous magnetic field may be a time-varying magnetic field at the same location as an imaging plane of an ultrasound transducer wherever the probe is positioned spatially relative the imaged subject. Magnetic nanoparticles, for example made from magnetite ($Fe_3O_4$), maghemite or zinc-doped magnetite, and usually with a size range of 10-100 nm (incl coating), are located at the target location within the imaging plane. Alternatively, magnetic microparticles or contrast agents made from bubbles coated with magnetic material may be used.

The time-varying magnetic field (T) is provided at a target location in the imaging plane of the ultrasonic transducer. Magnetic nanoparticles that are located at the target location in the imaging plane thus exhibit spatial fluctuations in the time-varying magnetic field (T) and are therefore forced to translate, displace, and/or rotate under the influence of the magnetic field (T). Because of the creation of the time-varying magnetic field component in the axial direction of the imaging plane, the displacement amplitude of the nanoparticles and their surrounding can be detected by the ultrasonic transducer. The time varying magnetic field may have a gradient which may cause the nanoparticles to translate, displace and/or rotate under the influence of the magnetic field (T). Therefore, the translatory or displacement motion of the particles may be oscillating due to the time variation of the magnetic field.

The shape of the particle may further enhance or suppress the action of translation, displacement, and/or rotation. For example, particles having an oval/elliptic shape may give rise to a rotational movement which together with a translational movement may be detectable with the ultrasound transducer and could be used to improve the detection. Spherical particles may also rotate due to the gradient of the time-varying magnetic field. Further, other shapes, such as rod shaped, octagonal or star shaped particles may also give rise to movements which may be used to improve the detection.

Alternatively, and/or additionally, lateral motion detection may also be possible.

By observing the movement and displacement of the nanoparticles in the imaging plane an accurate representation of the concentration can be found which could be used to outline an object in the magnetomotive image or spots in a tissue with increased nanoparticle concentration.

The displacement of the nanoparticles may also be dependent on the distance between the magnet and where in the imaging plane the nanoparticles are positioned.

The motion, such as a vibration, that appears in the tissue where particles are present can be detected with ultrasound and processed by software, which filters, enhances and then visualizes the image generating magnetomotive signal.

For diagnostic purposes, one is typically interested in the concentration of the contrast agent in the tissue. Under reasonable conditions the concentration of the contrast agent is approximately proportional to the amplitude of the oscillating movement induced by the nanoparticles.

Note that the amplitude of the oscillation is typically very small - on the scale of micrometers, see Maria Evertsson et al, "Multimodal detection of iron oxide nanoparticles in rat lymph nodes using magnetomotive ultrasound imaging and magnetic resonance imaging", IEEE transactions on ultrasonics, ferroelectrics, and frequency control, 61(8):1276-1283, 2014.

This makes the movement impossible to observe in a standard B-mode ultrasound image. Instead, one seeks to construct an image where each pixel encodes the amplitude and/or phase of the oscillating movement at the corresponding location.

Under the assumption that the oscillation is sinusoidal and reasonably stationary, the amplitude and phase can conveniently be represented by a single complex number called the complex amplitude of the oscillating movement. In the imaging pipeline of a magnetomotive ultrasound system, one seeks to convert the raw radio frequency (RF) data from the ultrasound device to a sequence of complex valued images, where each pixel describes the amplitude and/or phase of the tissue displacement at the given time and location.

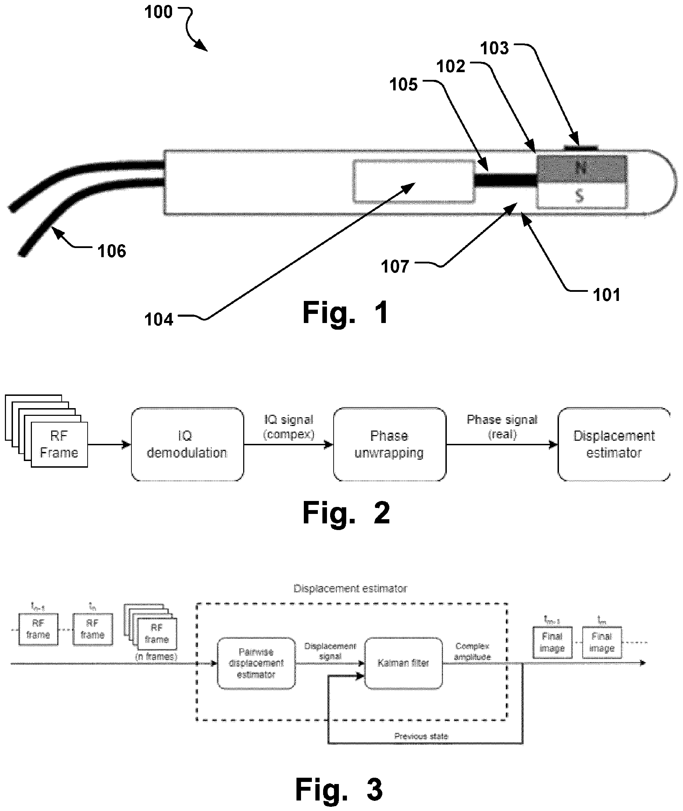

Fig. 1 is illustrating a schematic magnetomotive imaging probe device 100. The probe device is described in WO 2022/049297, which is incorporated by reference in its entirety. The device includes a housing 101 having an outer surface and an inner cavity 107. The device may further include a magnet 102 arranged in the inner cavity 107 of the housing 101. The magnet 102 is configured for generating a time-varying magnetic field. The magnet 102 may be a cylindrically shaped permanent magnet, preferably a diametrically magnetized magnet. Such a diametral magnet is a cylindrically shaped magnet, having a rotational axis extending along a symmetry axis of the cylindrically shaped magnet. This type of magnet provides a magnetic field orthogonally to the rotation axis. In some example may the shape of the magnet be a rectangular cuboid, such as a cube, rotating around an axis and provides a magnetic field orthogonally to the axis, similar as for a diametral magnet. The magnet has opposite magnetic poles (N, S) separated along a diameter of the magnet in the radial direction (r), whereby rotation of the magnet, create a time-varying magnetic field (T), at a target location. It is conceivable that other shapes of the magnet 102, may provide the same effect.

The time-varying magnetic field may be obtained by rotating the magnet 102 using a motor 104. The magnet 102 may be rotated by the motor 104 via a shaft 105.

Alternatively, in some examples may an electric magnet be used, and the time-varying magnetic field may be obtained by switching the poles, driving a sinusoidal current, or any time-varying current that results in the desired time variation of the magnetic field.

For detecting the translation displacement and/or rotation of the particles, a distance, movement, or magnetic material sensing device 103, for example an ultrasound transducer, such as an ultrasound array, an optical sensor (for example laser Doppler), a susceptometer, or other means for detecting distance, movement, or magnetic material known to the person skilled in the art, may be used. For the rest of this application, an ultrasound transducer will be used as an example of a sensing device, but as described other types of sensing devices could be used instead.

The ultrasound transduced may be arranged on the outer surface of the housing 101 so that an imaging plane of the ultrasound transducer 103 is within the time-varying magnetic field. In particular, this may be achieved by arranging an ultrasound transducer 103 on an outer surface of the housing 101 so that the ultrasound transducer 103 is positioned along a length of the magnet 102. Preferably a length of the cylindrical magnet 102 exceeds a width of the ultrasound transducer 103. The ultrasound transducer 103 will then be positioned within the time varying magnetic field together with the imaging plane of the ultrasound transducer 103. This arrangement may increase the sensitivity of detection of magnetic nanoparticles inside the volume of the magnetic field moving due to the variation of the magnetic field.

Typically, ultrasound pulses in the geometric shape of a beam, are fired using a number of the elements in the array, whereby a focused beam may be achieved by delaying the transmission of certain elements such that the sound interferes constructively at the focus. Likewise, the returning echoes may be detected on a number of elements, and a reconstructed signal may be achieved such that echoes along the transmit beam are amplified, by the same delays as in the transmit case. The received RF data from all transmitted beams are then beamformed and mapped onto an image with geometrical shape that corresponds to the area covered by all transmitted beams and/or beamformed received RF data. This includes a geometrical transformation as well as interpolation between beams in a process often referred to as scan-conversion.

Alternatively, a plane wave can be transmitted using a number of the elements in the array. The receiving echoes may be detected on a number of elements. The received RF data from the plane wave may be beamformed and mapped onto an image with geometrical shape that corresponds to the area covered by the transmitted plane wave and/or beamformed received RF data. This includes a geometrical transformation as well as interpolation between beams in a process often referred to as scan-conversion.

Beamforming of the received RF data may be done either before or after the herein described algorithm. In the described examples, beamforming is performed before performing the algorithm and the algorithm is performed before the beamformed data is mapped to an image with a geometrical shape (scan-conversion). Alternatively, the algorithm may be applied on beamformed data that has been scan converted.

Typically, the magnetic particles are much smaller than the resolution cell of the ultrasound, but their displacement upon the action of the magnetic field, may create a detectable motion in their immediate surrounding, such that the motion can be detected by ultrasound. This can be done by covering the imaging plane by imaging beams at a rate exceeding that of the magnetic field frequency. Theoretically the rate of imaging (probing) at one location must be at least twice that of the induced motion of the nanoparticles, but practically even higher, 4-10 times for instance. Other imaging schemes are possible, such as creating a plane wave insonifying a large portion of the region to be imaged and in retrospect perform receive focusing on the received echo data stored in a memory.

The sensitivity may be further enhanced by arranging the magnet 102 and the ultrasound transducer 103, in such a way that the active ultrasound elements are situated as close as practically possible to the magnet 102. When the ultrasound transducer is arranged on the housing, as illustrated in Figs. 1, a distance between the magnet 102 and the ultrasound transducer 103 may be defined mainly by the thickness of the material of housing 101 at the position of the ultrasound transducer and/or a distance between an inner surface of the housing and the magnet 102.

The probe 100 may further include connectors 106, such as cables, for controlling the probe and for transferring recorded data, such as imaging data from the ultrasound transducer 103 to a computer or a control unit which includes a processor. Additionally, and/or alternatively, the probe may be controlled, and the data may be transferred wirelessly, such as over Wi-Fi or other types of wireless protocols, such as Bluetooth.

Additionally, and/or alternatively, the cables 106 may be used for providing power to the probe. Alternatively, the power to the probe may be provided using batteries arranged in the probe.

The motor 104 of the magnetomotive imaging probe device 100 may be controlled by a control unit (not shown). As described above, the motor 104 may be coupled to the control unit via the connectors 106 or through a wireless protocol. The power of the motion of the magnet 102 may then be controlled. The control unit may be adapted to vary the speed of motion (w) of the magnet 102, according to a predetermined pattern to thereby vary the frequency of the time-varying magnetic field (T) as a predetermined impulse to generate an impulse response of magnetic nanoparticles at the target location. This provides for determining an impulse response from the nanoparticles that may be indicative of the material properties, such as viscosity, elasticity and density. Hence, the nanoparticles may be displaced by the magnetic impulse and the material properties will affect how the displacement varies over time, such as the dominant frequency, maximum amplitude, and speed of damping may be indicative of material density, the elasticity and viscosity.

The control unit may be adapted to vary the speed of motion (w) of the magnet 102, such as by increasing the speed linearly up to a certain maximum speed, and thereafter decrease the speed, to provide a sweep throughout frequencies (chirp) and detect the resulting displacement amplitude of the nanoparticles. The control unit may thus be adapted to vary the speed of motion (w) of the magnet 102, according to such predetermined pattern to provide for detection of a frequency impulse response. The control unit may be adapted to set a constant speed of motion (w) of the magnet 102. Depending on the position of the S-pole relative the N-pole during rotation of the magnet the rotational force may vary, for instance due to influence of the earth magnetic field or iron structures in the proximity, which thus may be compensated by the control unit.

The control unit may be further adapted to synchronize the frequency or speed of motion (w) of the magnet 102, to the ultrasound imaging in order to provide for ultrasound detection at the magnetic field frequency (that of the magnetic field time-variation), and further to allow for detection at the phase of the magnetic field relative the ultrasound imaging.

The ultrasound transducer 103 may also have an ultrasound control unit (not shown) that provides for the necessary control and analysis related to the ultrasound equipment.

The device may include different sensors, for example a sensor for tracking the position of the magnet during the rotation. This could be an encoder for detecting at least one pulse on each rotation. This may be useful for synchronizing the frequency or speed of motion (w) of the magnet 102, to the ultrasound imaging or when having a gearbox, such as reduction gear. Other sensors that could be used are, an accelerometer to detect if the device is held steady or is moving, a gyroscope, thermometer, a pH sensor, a sensor for detecting interfering magnet fields.

The housing should be made of a non-electrically conductive material to avoid eddy currents which may reduce the delivered magnetic field. One such material is Polyether ether ketone (PEEK). Other materials could be Polyphenylene sulfide (PPS), or polysulfone.

Raw radio frequency (RF) data from the ultrasound transducer may be converted to a sequence of complex valued images by a control unit or a data processing device connected to the magnetomotive ultrasound imaging device or be saved and converted later.

All determinations or calculations described herein may be performed by a control unit or a data processing device (not illustrated). The data processing device should not be understood as limited to a single device carrying out all steps, but could be a plurality of data processing devices wherein each processing device carries out a part of the method. The control unit or a data processing device may be implemented by special-purpose software (or firmware) run on one or more general-purpose or special-purpose computing devices. In this context, it is to be understood that each "element" or "means" of such a computing device refers to a conceptual equivalent of a method step; there is not always a one-to-one correspondence between elements/means and particular pieces of hardware or software routines. One piece of hardware sometimes comprises different means/elements. For example, a processing unit serves as one element/means when executing one instruction, but serves as another element/means when executing another instruction. In addition, one element/means may be implemented by one instruction in some cases, but by a plurality of instructions in some other cases. Such a software controlled computing device may include one or more processing units, e.g. a CPU ("Central Processing Unit"), a GPU ("Graphic processing unit"), a DSP ("Digital Signal Processor"), an ASIC ("Application-Specific Integrated Circuit"), discrete analog and/or digital components, or some other programmable logical device, such as an FPGA ("Field Programmable Gated Array"). The data processing device may further include a system memory and a system bus that couples various system components including the system memory to the processing unit. The system bus may be any of several types of bus structures including a memory bus or memory controller, a peripheral bus, and a local bus using any of a variety of bus architectures. The system memory may include computer storage media in the form of volatile and/or non-volatile memory such as read only memory (ROM), random access memory (RAM) and flash memory. The special purpose software may be stored in the system memory, or on other removable/non-removable volatile/non-volatile computer storage media which is included in or accessible to the computing device, such as magnetic media, optical media, flash memory cards, digital tape, solid state RAM, solid state ROM, etc. The data processing device may include one or more communication interfaces, such as a serial interface, a parallel interface, a USB interface, a wireless interface, a network adapter, etc., as well as one or more data acquisition devices, such as an A/D converter. The special-purpose software may be provided to the control unit or data processing device on any suitable computer readable medium, including a record medium and a read-only memory.

The simplest and most studied technique for estimating complex amplitude given RF data is described in Maria Evertsson, "Development of Magnetomotive Ultrasound Imaging", PhD thesis, Lund University, 2016; Sandra Sjöstrand et al, "Magnetomotive ultrasound imaging systems: basic principles and first applications", Ultrasound in Medicine & Biology, 46(10):2636- 2650, 2020; and Maria Holst et al, "Phase-locked magnetomotive ultrasound imaging of superparamagnetic iron-oxide nanoparticles", 2010 IEEE International Ultrasonics Symposium, pages 1007-1010. IEEE, 2010. The method can be described as comprising:

1. Perform IQ demodulation of the RF data with respect to the ultrasound carrier frequency.
2. Extract the phase of the IQ signal (phase unwrapping may be necessary). Doing this for several consecutive ultrasound frames gives a signal constituting of a series of phase values, referred to as the displacement signal, proportional to the displacement represented at each pixel's location.

3. Estimate the complex amplitude of the displacement signal. This could, for instance, be done through least-squares sinusoid fitting to a periodic reference signal with oscillation frequency equal to that of the nanoparticles. Another option is to perform an additional IQ demodulation of the displacement signal with respect to the nanoparticle frequency.

A flowchart of the algorithm for the phase-shift method for estimating displacement is illustrated in Fig. 2. This method uses the phase signal directly as the displacement signal.

The major drawback with this method is that the phase of the IQ signal in step 2 above tends to be noisy. To compensate for this, one needs a large number of frames, typically hundreds, to estimate the complex amplitude in step 3 above and get a stable image. This results in high amounts of latency, which makes the method inherently hard to use together with hand-held ultrasound devices.

As mentioned in the introduction, nanoparticle displacement is typically at the sub-pixel level. This means that any motion of the ultrasound image large enough to result in pixels changing place will obscure the signal, effectively making it impossible to extract the sub-pixel nanoparticle displacement from a batch of frames. In general, any motion of the ultrasound device during frame acquisition will to some degree add noise to the displacement signal.

There are also less noisy methods described in the literature. In Thomas Ersepke et al, "On the performance of time domain displacement estimators for magnetomotive ultrasound imaging", IEEE transactions on ultrasonics, ferroelectrics, and frequency control, 66(5):911-921, 2019, several techniques for estimating displacement between pairwise frames are described and evaluated. Specifically, three other methods are mentioned:

1. A method based on minimizing normalized cross correlation (NCC) between frames.
2. A Bayesian method including line-by-line regularization.
3. A method called GLUE (Global ultrasound elastography) which is basically identical to the classical method by Horn-Schunck for estimating optical flow, Berthold KP Horn et al, "Determining optical flow", Artificial intelligence, 17(1-3):185-203, 1981.

In order to improve and extend the use of magnetomotive ultrasound imaging, such as making a hand-held ultrasound device or visualization of moving tissue, for example movement of a patient, possible, the time window during which each batch of frames is acquired needs to be kept to a minimum.

To make this possible, a new method is proposed. In the proposed method, the time window during which each batch of frames is acquired is kept to a minimum by in every step of the computation including the solution from the previous time step as prior.

Through frame-by-frame integration, any of the techniques described in Thomas Ersepke et al, "On the performance of time domain displacement estimators for magnetomotive ultrasound imaging", IEEE transactions on ultrasonics, ferroelectrics, and frequency control, 66(5):911-921, 2019 for displacement estimation between pairwise frames could be used repeatedly to replace the first two steps in the method described by Maria Evertsson, "Development of Magnetomotive Ultrasound Imaging", PhD thesis, Lund University, 2016; Sandra Sjöstrand et al, "Magnetomotive ultrasound imaging systems: basic principles and first applications", Ultrasound in Medicine & Biology, 46(10):2636-2650, 2020; and Maria Holst et al, "Phase-locked magnetomotive ultrasound imaging of superparamagnetic iron-oxide nanoparticles", 2010 IEEE International Ultrasonics Symposium, pages 1007-1010. IEEE, 2010.

Below is a high-level description of the imaging pipeline provided. The pipeline produces images in real-time in an on-line fashion. The input is a sequence of RF data frames which are consumed by the pipeline in batches at each time step. The batches may overlap. For example, the batch may be a single frame from the sequence of RF data frames, or at least two frames from the sequence of RF data frames. The two or more frames may not immediately following each other, thus there may be frames in between which are not used. Should the batch include more than 2 frames, overlapping between batches may be possible. For example, where the last frame(s) of a first batch overlaps with the first frame(s) of a second batch and the last frame(s) of the second batch overlaps with the first frame(s) of a third batch and so on.

The output is a sequence of images describing the complex amplitude. This can now be represented and combined with the B-mode image in various ways in order to present the information to the user.

Two variants of the proposed method are described. The first variant, called "two-step approach", estimates the complex amplitude in a two-step fashion, whereas in the second variant, called "one-step approach", the complex amplitude is estimated directly in one single computational step.

The high-level description of the pipeline may include the following steps:

1. At each time step, a mini batch of the last RF data frames are selected.
2. In addition, the solution from previous time step together with an estimate of the error, referred to as the precision matrix, is used as prior.
3. "**Two-step approach**": The displacement signal may be extracted, for instance using NCC or optical flow followed by frame-by-frame integration.

"**One-step approach**": Explicit extraction of the displacement signal is skipped.

4. "**Two-step approach**": The complex displacement amplitude at the nanoparticle oscillation frequency may, for instance, be estimated using recursive least-squares fitting in the form of a Kalman filter, Greg Welch, et al., "An introduction to the Kalman filter", 1995. "One-step approach": If using optical flow for pairwise displacement estimation, another possibility may be to instead estimate the displacement amplitude directly in step 3 by adding additional constraints to the functional minimized when estimating the optical flow, see section below related to the "one step approach".

5. The solution can be presented to the user in various ways, for instance in the form of a heat map showing the displacement amplitude and/or phase for each pixel. In addition, by applying some form of thresholding to the solution, the heat map can be put on top of the B-mode image as an overlay. Heat map is a graphic representation of data where values are depicted by colour.

6. The solution is now fed as prior to the next step of the algorithm. Initially, for the first batch of frames, the estimated previous state can be set arbitrarily, for instance as the identity matrix or just to random numbers, and just setting the initial *precision matrix* (see section below related to "one-step approach") to a low precision, for example a null matrix.

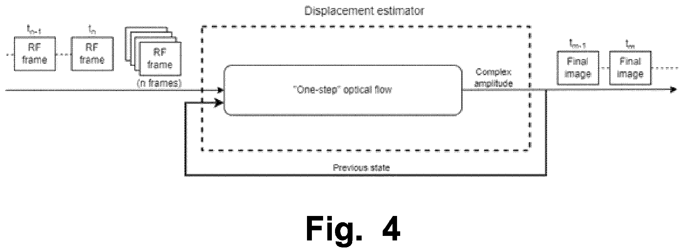

In both variants of the algorithm, the displacement estimator (see Fig. 3 & 4) is designed with the following goals in mind:

a) The solution should be consistent with the batch of RF data frames under the assumption that the situation is stationary (nanoparticles oscillating at a constant frequency).
b) Solutions near the prior solution should be preferred if the prior is deemed accurate enough.
c) The solution should be smooth in the sense that it does not vary much in a small region.
d) The minimization problem should be feasible to solve in real-time.

Note that the stationary assumption in the first goal might not be accurate if the ultrasound equipment is moved, for instance during hand-held operation. The algorithm can be augmented with motion detection. Interfering or artefactual motion may be detected by comparing a sub-batch with another sub-batch. A sub-batch could include a single frame or more. When carrying out the comparison, an average of the frames in each batch may be compared. The sub-batches could be picked from any position in a batch of frames and the two sub-batches to be compared does not have to be consecutive without interruption of frames in between. For example, the last two RF data frames in the batch at the beginning of each computation. In this case, each sub-batch would include a single frame. If the absolute difference between these two RF data frames is greater than a pre-defined threshold, motion is detected. If not, motion is not detected. If motion is detected the precision matrix is set to zero at the start of the next computation, thereby removing the constraint on similarity between new and previous solutions (see step b).

An alternative to the above motion detection may be to use a sensor arranged in the probe, such as an accelerometer.

By interpreting the precision matrix as measuring uncertainty or quality of the underlying IQ signal, the precision matrix can be used to estimate the significance of a given displacement estimation from the algorithm. Other means of measuring the signal quality could be for instance to measure the signal-to-noise ratio or the spatial variance. For instance, pixel displacements, such as displacement estimation at the location of an image pixel, with significance values lower than some pre-defined threshold could be set to zero, and instead display the grayscale B-mode value.

With some adjustments, the algorithm could be used to estimate displacement parameters for non-stationary oscillations as well, e.g. shorter pulses, chirps or ramped sinusoids. Estimations of the quality of the underlying IQ signal, for instance the signal-to-noise ratio or the spatial variance, could also be used to help determine the significance of a given solution.

Two-step approach

Fig. 3 illustrates a flowchart of the two-step version of the algorithm. The complex amplitude may be estimated recursively using a Kalman filter, where the previous state (complex amplitude) together with an estimate of its accuracy is used as prior at each time step.

One way of incorporating prior knowledge when estimating the complex amplitude is to use a Kalman filter (see Fig. 3). In this section we give an example of how the displacement estimation can be done using optical flow. Note however that any method for pairwise displacement estimation could be adopted. In this variant, for two RF data frames $f^1$ and $f^2$, the optical flow can be found by minimizing the following functional with regards to an arbitrary displacement field $u(x,y)$:

$$E(u) = \int_{\Omega} \left( \left( f^1(x, y + u(x,y)) - f^2(x,y) \right)^2 + \alpha_x |u_x|^2 + \alpha_y |u_y|^2 \right) \mathrm{d}x \mathrm{d}y, \quad (1)$$

where $\alpha_x$ and $\alpha_y$ are empirically determined regularization parameters, and $u_x$ and $u_y$ are the spatial partial derivatives of $u$. These terms add a constraint on smoothness to the solution, Berthold KP Horn et al., "Determining optical flow", Artificial intelligence, 17(1-3):185-203, 1981. In Eq. 1 the nanoparticles are assumed to oscillate in the $y$-direction. $\Omega$ is the set of points (x, y) on which the image is defined.

A detailed description of how to minimize $E(u)$ with respect to $u$ is given in the section below about "One-step approach".

Without having, for example to maintain a window buffer or specifying a particular window size, recursive least-squares fitting in the form of a Kalman filter may be used. The state of the filter is taken to be the in-phase and quadrature amplitudes $A$ and $B$ of the displacement signal. The predicted displacement given the filter state is then given by:

$$y(t) = A \cdot cos(\omega t) + B \cdot sin(\omega t).$$

This model may be used for sinusoidal fitting, for other forms, such as chirp frequency or other types of frequency modulation, other models may have to be used for the predicted displacement. It may further be synchronized with, or related to, the phase of the magnetic force at the point of interest in the tissue resulting from the magnet in the probe. The phase could either be the phase of an electromagnet or the movement, such as a rotation, of a permanent magnet.

The state is assumed to be stationary. The measurement noise covariance, as discussed in Greg Welch et al., "An introduction to the Kalman filter", 1995, may be set to an appropriate constant value, or estimated based on e.g. the optical flow residual error or the filter prediction error from previous iterations. The ability to easily control the process noise covariance is advantageous; for example, the signal integration history can quickly be erased if it is invalidated by probe motion, by simply raising the value when motion is detected. When the probe is still, longer integration times and lower noise can be obtained by lowering the value.

One-step approach

Fig. 4 illustrates a flowchart of an example of the one-step version of the algorithm. By incorporating time dependency already in the optical flow step, the complex amplitude may be estimated directly from a sequence of RF data frames in an "end-to-end" fashion. At every time step, the previous state (complex amplitude) together with an estimate of its accuracy is used as prior. For example, initially no previous state exists, whereas this part is skipped by initializing the precision matrix $\Sigma^{-1}$ to zero (see Eq. 2).

In the section about "two-step approach", the complex amplitude was estimated in a two-step fashion. In the first step, when estimating the optical flow, an arbitrary displacement field $u(x, y)$ was employed. By incorporating the knowledge of what signal we are really looking for already in this step we can get an estimation of the displacement parameters directly, without the need for subsequent least-squares fitting.

The sequence of RF data frames is now interpreted as a time dependent two-dimensional image $f(x, y, t)$. The image is assumed to oscillate in, say, the $y$-direction such that it is displaced by a sinusoidal function with known frequency but unknown phase and amplitude.

Mathematically, this can be modelled as

$$f(x, y, t) = f^{\text{ref}}(x, y - A \cdot cos(\omega t) - B \cdot sin(\omega t), t) + N\left(0, \sigma(x, y)\right),$$

where $f^{\text{ref}}$ is a hypothetical reference image and $N$ is a noise term.

Now, let $f(x, y, t_k)$, $k = 1, \ldots, n$ be a sequence of frames and define:

$$\tilde{f} = \frac{1}{n} \sum_{k=1}^{n} f^k, \qquad f^k = f(x, y + e_k \cdot u, t_k),$$

$$e_k = (cos(\omega t_k), sin(\omega t_k)), \qquad u(x, y, t_k) = \left( A(x, y, t_k), B(x, y, t_k) \right).$$

Note that $\tilde{f}$ is a reasonable estimate of $f^{\text{ref}}$ provided that $u$ is a good estimate of the optical flow. In particular, the quantity

$$\textstyle\sum_{k=1}^{n}\left(f^{k}-\tilde{f}\right)^{2}$$

measures the error given a hypothetical optical flow $u$. Note that this is just one of many possible choices for error estimation. For instance, one could also use the deviation from the median $\sum_{k=1}^{n}\left(f^{k}-\bar{f}\right)^{2}$, or the successive error $\sum_{k=1}^{n}(f^{k}-f^{k-1})^{2}$.

Adding regularization terms, and assuming an *a priori* solution $u^{\mathrm{p}}$ with covariance $\Sigma$, the complete algorithm then boils down to minimizing the following functional:

$$E(u) = \int_{\Omega} \frac{1}{2} \left( \frac{1}{\sigma^2} \sum_{k=1}^{n} (f^k - \tilde{f})^2 + \alpha_x |u_x|^2 + \alpha_y |u_y|^2 \right.$$

$$+ (u - u^{\mathrm{p}})\Sigma^{-1}(u - u^{\mathrm{p}})^{\mathsf{T}} \Big) \mathrm{d}x \mathrm{d}y \, . \quad (2)$$

The precision matrix $\Sigma^{-1}$, determining the *significance* of the previous state $u^{\mathrm{p}}$, is computed as the inverse of the state covariance matrix of the integrand in Eq. 2, see William Carlisle Thacker, "The role of the hessian matrix in fitting models to measurements", Journal of Geophysical Research: Oceans, 94(C5):6177-6196, 1989; and Ken Binmore et al., "Calculus: concepts and methods", Cambridge University Press, 2001.

To minimize $E(u)$ with respect to $u$, we first linearize $f^k$ at $u = u^0$ and obtain:

$$f^k(u) \approx f^k(u^0) + f_y^k(u^0) \cdot e_k \cdot \Delta u \, .$$

By defining:

$$C_k = f_y^k(u^0) \cdot \cos(\omega t_k) - \frac{1}{n} \sum_{j=1}^{n} f_y^j(u^0) \cdot \cos(\omega t_j) \, ,$$

$$S_k = f_y^k(u_0) \cdot \sin(\omega t_k) - \frac{1}{n} \sum_{j=1}^{n} f_y^j(u^0) \cdot \sin(\omega t_j) \, ,$$

$$D_k = f^k(u^0) - \frac{1}{n} \sum_{j=1}^{n} f^j(u^0) \, ,$$

the right-hand side of Eq. 2 is approximated by:

$$\int_{\Omega} \frac{1}{2} \left( \frac{1}{\sigma^2} \sum_{k=1}^{n} \left( (C_k, S_k) \cdot \Delta u + D_k \right)^2 + \alpha_x |u_x|^2 + \alpha_y |u_y|^2 \right.$$

$$+ (u - u^{\mathrm{p}})\Sigma^{-1}(u - u^{\mathrm{p}})^{\mathsf{T}} \Big) \mathrm{d}x \mathrm{d}y \, . \quad (3)$$

Denoting the integrand by $L$, the Euler-Lagrange equations for this variational problem are:

$$\frac{\partial L}{\partial \Delta a} - \frac{\partial}{\partial x} \cdot \frac{\partial L}{\partial \Delta a_x} - \frac{\partial}{\partial y} \cdot \frac{\partial L}{\partial \Delta a_y} = 0\,,$$

$$\frac{\partial L}{\partial \Delta b} - \frac{\partial}{\partial x} \cdot \frac{\partial L}{\partial \Delta b_x} - \frac{\partial}{\partial y} \cdot \frac{\partial L}{\partial \Delta b_y} = 0\,.$$

Applying this to Eq. 3 gives the system:

$$\frac{1}{\sigma^2}\left(\begin{pmatrix} C_k^2 & C_k S_k \\ C_k S_k & S_k^2 \end{pmatrix}\Delta u + D_k \begin{pmatrix} C_k \\ S_k \end{pmatrix}\right) + \Sigma^{-1}(u^0 + \Delta u - u^p)$$
$$= \alpha_x(u_{xx}^0 + \Delta u_{xx}) + \alpha_y\left(u_{yy}^0 + \Delta u_{yy}\right).$$

By discretising the differential operators, we get a sparse linear system which we can solve for $\Delta u$ using for instance an iterative method such as successive over-relaxation [10, 11]. Iterating the whole process until convergence, using $u^0 + \Delta u$ as our new $u^0$, we get a solution for $u$.

As mentioned, the absolute value of the complex amplitude is taken as the displacement of particles (or tissue complex containing particles). But the phase (or argument of the complex amplitude) may also contain important information, such as precise location of nanoparticles or information about tissue stiffness. Consider for instance nanoparticles attracted by a rotating magnet as described above. The particles will move in synch with the magnetic field (possibly with some small delay due to viscoelastic properties of the tissue). Since tissue is cohesive, the motion will to some degree be transferred to adjacent tissues, and the detected motion may then be interpreted as presence of nanoparticles. But as the motion of the tissue is transferred with a delay due to the wave propagation velocity, the phase of the displacement will be different in the regions not containing nanoparticles. By imposing a limit on the phase for the display of the displacement value, a more precise detection of the location of nanoparticles or estimated tissue stiffness may be obtained. Furthermore, by identifying the location of the center of the displacement amplitude induced by the nanoparticles, knowledge of the transferred movement which gradually decays with distance from the origin could be filtered out, further enhancing the precision in the localization of nanoparticles. Normally, the location of the center of the displacement amplitude induced by the nanoparticles is the location of the highest amplitude. By identifying a local maximum in the displacement amplitude and consider as for example deducting the expected decay in displacement amplitude around the maximum which is caused by the spreading movement one can obtain a more precise image of the actual movement origin and thereby nanoparticle location.

The velocity by which the wave propagates is also dependent on the viscoelastic properties. The phase may therefore also be an important marker for estimating tissue mechanical properties. Non-limiting examples of tissue mechanical properties may be, for example, elasticity, viscosity or Poisson's number.

The method step described herein may be incorporated as a software, computer program or as a computer implemented invention.

The present disclosure has been described above with reference to specific embodiments. However, other examples than the above described are equally possible within the scope of the invention. Different method steps than those described above may be provided within the scope of the invention. The different features and steps of the invention may be combined in other combinations than those described. The scope of the disclosure is only limited by the appended patent claims.

More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present invention is/are used.

## Claims

1.  A method for estimating at least one displacement parameter, characterizing a displacement signal of a medium in a sequence of ultrasound data frames, such as ultrasound RF data frames, comprising

    acquiring a first batch of data frames using an ultrasound device;

inputting the first batch of data frames into a displacement parameter estimating algorithm together with an estimated previous state to obtain an estimated next state, the estimated next state comprising an amplitude and a phase, and

wherein the estimated next state is used as the estimated previous state for a second batch of data frames; and associate the at least one displacement parameter estimated by said estimating algorithm with at least one of the amplitude and the phase; and

using the at least one displacement parameter to localize movement in the medium.

2. The method of claim 1, wherein a fitting model is used to localize the movement in the medium, such as a sinusoidal fitting model.

3. The method of any of claims 1 to 2, wherein the ultrasound device is part of a Magnetomotive ultrasound imaging device and/or a device for elastography.

4. The method of any of claims 1 to 3, wherein the amplitude and the phase are represented by a complex amplitude.

5. The method of any of claims 1 to 4, coding the phase and the amplitude estimated by said estimation algorithm using a heat map and scan conversion to provide an output image.

6. The method of any of claims 1 to 5, wherein a precision matrix is obtained for determining the significance of the previous state; or
wherein a precision matrix is used for estimating the significance of a displacement estimation; or wherein a precision matrix is used for estimating the significance of a displacement estimation being a displacement estimation at the location of an image pixel, wherein the displacements with significance values lower than a pre-defined threshold is set to zero, optionally, the pixels set to zero displays a grayscale B-mode value.

7. The method of any of claims 1 to 6, wherein the second batch of data frames comprises at least one data frame acquired subsequent the data frames of the first batch, or a combination of at least one data frame of the first batch together with at least one data frame acquired subsequent the data frames of the first batch.

8. The method of any of claims 1 to 7, wherein for the first batch of data frames, the estimated previous state comprises at least one displacement parameter set to a predefined value and the value representing the accuracy of the estimate of the at least one displacement parameter is set to define the accuracy as either high or low.

9. The method of any of claims 1 to 8, wherein the displacement represents movement induced by particles in magnetomotive ultrasound imaging.

10. The method of any of claims 1 to 9, wherein the data frames of said first and second batch are subsequently ordered.

11. The method of any of claims 1 to 10, wherein the displacement estimating algorithm comprises a pairwise displacement estimator and a recursive least-squares fitting for estimating the amplitude and the phase, and wherein the previous state together with an estimate of its accuracy is used as prior for each calculation using the displacement parameter estimating algorithm.

12. The method of any of claims 1 to 10, wherein the displacement estimating algorithm comprises an optical flow step and the amplitude and the phase is estimated directly from the first batch of data frames by including a time dependency in the optical flow step, and at every time step during which the first batch is acquired, the previous state together with an estimate of its accuracy is used as prior.

13. The method of any of claims 1 to 12, detecting interfering or artefactual motion by comparing said first batch with said second batch.

14. The method of any of claims 1 to 13, wherein the phase is used for estimating a location of the nanoparticles or providing information about tissue stiffness, such as a detected motion of tissue is interpreted as presence of nanoparticles and since the motion is transferred in tissue with a delay, the phase is different in regions not containing the nanoparticles, whereby the difference in phase is used for detecting the location of the nanoparticles.

15. A computer program comprising instructions which, when the program is executed by a computer, cause the computer to carry out the method of any of claims 1 to 15.

16. A system for examining tissue, comprising:

a magnetomotive ultrasound device; and
a processing unit configured to execute the computer program of claims 1 to 14.

**Fig. 1**

**Fig. 2**

**Fig. 3**

**Fig. 4**

Europäisches
Patentamt

European
Patent Office

Office européen
des brevets

## EUROPEAN SEARCH REPORT

Application Number

EP 23 21 9558

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2008/021945 A1 (HAMILTON JAMES [US] ET AL) 24 January 2008 (2008-01-24) | 1-11, 13-16 | INV. A61B8/08 |
| Y | * paragraphs [0010], [0013], [0015], [0016], [0019]; figures 1,2,3,4,5 * | 12 | G01S7/52 G06T7/00 |
| X | CN 111 265 250 A (NEUSOFT MEDICAL SYSTEMS CO LTD) 12 June 2020 (2020-06-12) * paragraph [0031] - paragraph [0207]; figures 1-9 * | 1,15,16 | |
| Y | PELLOT-BARAKAT C ET AL: "ULTRASOUND ELASTOGRAPHY BASED ON MULTISCALE ESTIMATIONS OF REGULARIZED DISPLACEMENT FIELDS", IEEE TRANSACTIONS ON MEDICAL IMAGING, IEEE, USA, vol. 23, no. 2, 1 February 2004 (2004-02-01), pages 153-163, XP001238183, ISSN: 0278-0062, DOI: 10.1109/TMI.2003.822825 * page 153 * | 12 | |
| A | KRANEMANN TIM C ET AL: "Real-Time Magnetomotive Ultrasound Imaging Using a Recursive Estimator", 2018 IEEE INTERNATIONAL ULTRASONICS SYMPOSIUM (IUS), IEEE, 22 October 2018 (2018-10-22), pages 1-4, XP033479843, DOI: 10.1109/ULTSYM.2018.8579643 [retrieved on 2018-12-17] * the whole document * | 1-16 | TECHNICAL FIELDS SEARCHED (IPC) A61B G01S G06T |
| A | US 2023/293152 A1 (EVERTSSON MARIA [SE] ET AL) 21 September 2023 (2023-09-21) * the whole document * | 1-16 | |

The present search report has been drawn up for all claims

| Place of search | Date of completion of the search | Examiner |
|---|---|---|
| Munich | 12 June 2024 | Marzal-Abarca, X |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 21 9558

This annex lists the patent family members relating to the patent documents cited in the above-mentioned European search report.
The members are as contained in the European Patent Office EDP file on
The European Patent Office is in no way liable for these particulars which are merely given for the purpose of information.

12-06-2024

| Patent document cited in search report | | Publication date | Patent family member(s) | | Publication date |
|---|---|---|---|---|---|
| US 2008021945 | A1 | 24-01-2008 | NONE | | |
| CN 111265250 | A | 12-06-2020 | NONE | | |
| US 2023293152 | A1 | 21-09-2023 | CN | 116249492 A | 09-06-2023 |
| | | | EP | 3964137 A1 | 09-03-2022 |
| | | | EP | 4188232 A1 | 07-06-2023 |
| | | | JP | 2023540570 A | 25-09-2023 |
| | | | US | 2023293152 A1 | 21-09-2023 |
| | | | WO | 2022049297 A1 | 10-03-2022 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2022049297 A

**Non-patent literature cited in the description**

- **MARIA EVERTSSON et al.** Multimodal detection of iron oxide nanoparticles in rat lymph nodes using magnetomotive ultrasound imaging and magnetic resonance imaging. *IEEE transactions on ultrasonics, ferroelectrics, and frequency control*, 2014, vol. 61 (8), 1276-1283
- **EVERTSSON M et al.** Frequency- and phase-sensitive magnetomotive ultrasound imaging of superparamagnetic iron oxide nanoparticles. *IEEE Trans Ultrason Ferroelectr Freq Control.*, March 2013, vol. 60 (3), 481-91
- Development of Magnetomotive Ultrasound Imaging. **MARIA EVERTSSON**. PhD thesis. Lund University, 2016
- **SANDRA SJÖSTRAND et al.** Magnetomotive ultrasound imaging systems: basic principles and first applications. *Ultrasound in Medicine & Biology*, 2020, vol. 46 (10), 2636-2650
- Phase-locked magnetomotive ultrasound imaging of superparamagnetic iron-oxidenanoparticles. **MARIA HOLST et al.** 2010 IEEE International Ultrasonics Symposium. IEEE, 2010, 1007-1010
- **THOMAS ERSEPKE et al.** On the performance of time domain displacement estimators for magnetomotive ultrasound imaging.. *IEEE transactions on ultrasonics, ferroelectrics, and frequency control*, 2019, vol. 66 (5), 911-921

- Phase-locked magnetomotive ultrasound imaging of superparamagnetic iron-oxide nanoparticles. **MARIA HOLST et al.** 2010 IEEE International Ultrasonics Symposium. IEEE, 2010, 1007-1010
- **THOMAS ERSEPKE et al.** On the performance of time domain displacement estimators for magnetomotive ultrasound imaging. *IEEE transactions on ultrasonics, ferroelectrics, and frequency control*, 2019, vol. 66 (5), 911-921
- **BERTHOLD KP HORN et al.** Determining optical flow. *Artificial intelligence*, 1981, vol. 17 (1-3), 185-203
- **GREG WELCH et al.** An introduction to the Kalman filter. *One-step approach*, 1995
- **GREG WELCH et al.** *An introduction to the Kalman filter*, 1995
- **WILLIAM CARLISLE THACKER**. The role of the hessian matrix in fitting models to measurements. *Journal of Geophysical Research: Oceans*, 1989, vol. 94 (C5), 6177-6196
- **KEN BINMORE et al.** Calculus: concepts and methods. Cambridge University Press, 2001